# EUROPEAN PATENT APPLICATION

(11) **EP 3 425 040 A1**
(43) Date of publication of application: **09.01.2019**
(21) Application number: 17760253.9
(22) Date of filing: 24.02.2017
(51) Int. Cl.: C12N 1/20, C12N 1/36, A61K 39/104, C12R 1/38

(54) **ATTENUATEDPSEUDOMONAS AERUGINOSA**

(30) Priority: 03.03.2016 KR 20160025963
(71) Applicant: Eyegene Inc., 401 Yangcheon-ro Gangseo-gu, Seoul 07528 (KR)
(72) Inventor: CHO, Yang Je, Seoul 04423 (KR); KIM, Kwangsung, Goyang-si Gyeonggi-do 10371 (KR); LEE, Na Gyong, Seoul 01913 (KR); PARK, Shin Ae, Seoul 07528 (KR)
(74) Representative: Gerbino, Angelo
(86) International application number: PCT/KR2017/002059
(87) International publication number: WO 2017/150849

(57) **Abstract**

The present invention relates to a novel attenuated *Pseudomonas aeruginosa* improved in productivity and safety and a vaccine composition for prevention of *P. aeruginosa* infection, comprising the same and, more particularly, to attenuated *P. aeruginosa* of Deposit Accession number KCTC 12901 BP, KCTC 12902BP, KCTC 12903BP, or KCTC 12904BP, and a vaccine composition for preventing *P. aeruginosa* infection, comprising the same.

## Description

### TECHNICAL FIELD

The present disclosure relates to a novel attenuated *Pseudomonas aeruginosa* strain having improved productivity and safety, a vaccine composition for preventing *Pseudomonas aeruginosa* infection including the attenuated *Pseudomonas aeruginosa* strain, and a method of preparing the attenuated *Pseudomonas aeruginosa* strain.

### BACKGROUND ART

*Pseudomonas aeruginosa* is an opportunistic infectious bacterium that is 1.5 µm to 3.0 µm in length and about 0.5 µm in width and has a single flagellum. *P. aeruginosa* is a Gram-negative motile bacterium that emits fluorescein which is a fluorescent pigment and pyocyanin which is a green pigment, and is widely distributed in soil, water, sewage, and the human gut [Mol. Microbiol. 4, 1069-1075, 1990].

*P. aeruginosa* is a pathogen that causes a variety of infectious diseases, for example, sepsis, systemic infections, chronic infections of the respiratory tract, intractable infections such as those in cystic fibrosis patients, etc. *P. aeruginosa* is a major pathogen that causes chronic pulmonary infection in patients with cystic fibrosis (CF), and is frequently found in patients with burn wounds, acute leukemia, or organ transplants, or in those who have received intravenous injections, in addition to patients with CF, and is also involved in external otitis, pneumonia, meningitis, urinary tract infections, and osteomyelitis (Kronberg, Lipopolysaccharide (LPS). 1-30, 1995). In particular, sepsis caused by *P. aeruginosa* is known as a disease caused by invasion of the microorganism itself or secretion of its toxic component into the blood of a patient whose resistance is decreased due to surgery, laceration, or trauma, resulting in shocks such as high fever, low blood pressure, etc., eventually leading to death. Further, *P. aeruginosa* has attracted more attention since it was also detected in the eyes of users of contact lens and in urinary tract infections.

Therefore, there has been a demand to develop drugs capable of effectively preventing or treating intractable purulent diseases such as sepsis, urinary tract infection, etc. which are caused by *P. aeruginosa,* and there are serious concerns about a high mortality rate due to *P. aeruginosa* infection, because *P. aeruginosa* has resistance against all but a few commercial antibiotics.

One of the practical treatments for *P. aeruginosa* infection is a method of neutralizing toxins by preparing antitoxins against *P. aeruginosa.* Antitoxins are therapeutic agents which can be used only in patients who have already developed sepsis, and antitoxins have disadvantages in that they have no prophylactic functions, and are fairly expensive, and thus they cannot be used universally in general patients. Further, the greatest drawback is that cure rates from treatment through administration of antitoxins are relatively low, and side effects are so severe that it is inappropriate to use antitoxins as therapeutic agents.

Another method of treating *P. aeruginosa* infection is to use broad spectrum antibiotics or chemotherapeutic agents having high selectivity against *P. aeruginosa.* However, since *P. aeruginosa* generally has very high drug resistance and many different types of *P. aeruginosa* exist, there have been many victim who have fallen to *P. aeruginosa* that could not be cured.

As another method, using cell fusion technology, a method of using monoclonal antibodies capable of neutralizing *P. aeruginosa* has been studied. However, this method is disadvantageous in that it cannot be used for prophylactic vaccines and it cannot treat all infections caused by the dozens of different *P. aeruginosa* strains.

In order to overcome these drawbacks, studies have been conducted to find a means which may exhibit excellent immunogenic (i.e., antigenic) reaction by effectively inducing the formation of antibodies against *P. aeruginosa* when administered into the human body, without the risk of inherent toxicity of *P. aeruginosa* itself.

Korean Patent No. 113196 suggests a method of obtaining an attenuated *P. aeruginosa* strain of which cell wall protein is useful for the preparation of prophylactic vaccines for *P. aeruginosa* infection, in which *P. aeruginosa* isolated from *P. aeruginosa*-infected patients is classified into 7 groups according to immunotype and each group is repeatedly attenuated. Subsequent studies were conducted to develop a therapeutic agent for *P. aeruginosa* infection including, as an active ingredient, human immunoglobulins specifically binding to outer membrane proteins which are isolated and purified from the *P. aeruginosa* strain. However, Korean Patent No. 113196 has a low yield of the attenuated strain and a safety risk due to high cytotoxicity.

Accordingly, the present inventors have made intensive efforts to develop *P. aeruginosa* that exhibits increased productivity and reduced release of toxins, as compared with the known methods, and as a result, have obtained a novel strain with improved productivity and safety due to remarkably reduced cytotoxicity resulting from reduced release of pathogenic factors, by selecting a colony among colonies obtained by culturing known *P. aeruginosa* and then repeatedly subculturing the colony, wherein i) the colony's size is relatively large, ii) its surface is not smooth, and iii) it does not form a clear zone in a culture medium containing skimmed milk, thereby completing the present disclosure.

### DESCRIPTION OF EMBODIMENTS

### TECHNICAL PROBLEM

An object of the present disclosure is to provide an attenuated *Pseudomonas aeruginosa* strain having improved productivity and safety.

Another object of the present disclosure is to provide a prophylactic vaccine composition including the attenuated *P. aeruginosa* strain.

Still another object of the present disclosure is to provide a method of preparing the attenuated *P. aeruginosa* strain.

### SOLUTION TO PROBLEM

In order to achieve the above objects, the present disclosure provides an attenuated *Pseudomonas aeruginosa* strain selected from the group consisting of Deposit Accession Nos. KCTC 12901 BP, KCTC 12902BP, KCTC 12903BP, and KCTC 12904BP.

Further, the present disclosure provides a vaccine composition for preventing *P. aeruginosa* infection, the vaccine composition including the attenuated *P. aeruginosa* strain.

Further, the present disclosure provides a method of preparing the attenuated *P. aeruginosa* strain, the method including repeatedly subculturing 40 to 60 times a strain selected from the group consisting of Deposit Accession Nos. KCCM 10029, KCCM 10030, KCCM 10031, and KCCM 10034.

### BRIEF DESCRIPTION OF DRAWINGS

FIG. 1 shows a comparison of cytotoxicity of a *P. aeruginosa* strain and a novel attenuated *P. aeruginosa* strain (KCTC 12901 BP);
FIG. 2 shows a comparison of cytotoxicity of a *P. aeruginosa* strain and a novel attenuated *P. aeruginosa* strain (KCTC 12902BP);
FIG. 3 shows a comparison of cytotoxicity of a *P. aeruginosa* strain and a novel attenuated *P. aeruginosa* strain (KCTC 12903BP); and
FIG. 4 shows a comparison of cytotoxicity of a *P. aeruginosa* strain and a novel attenuated *P. aeruginosa* strain (KCTC 12904BP).

### BEST MODE

Unless defined otherwise, all technical and scientific terms used herein have the same meanings as those generally understood by one of ordinary skill in the art to which the present disclosure belongs. Generally, the nomenclature used herein and the laboratory procedures described below are those well known and commonly employed in the art.

In the present disclosure, when a colony was selected from colonies obtained by culturing known *Pseudomonas aeruginosa* strain, wherein i) the colony's surface is not smooth, ii) a clear zone is not formed around the colony or a clear zone is less formed than those of other colonies, or iii) the colony's size is larger than those of other colonies, and then the selected colony was repeatedly subcultured, it was possible to obtain a novel strain having improved productivity and safety due to remarkably reduced cytotoxicity resulting from reduced release of pathogenic factors.

In an aspect, therefore, the present disclosure relates to an attenuated *P. aeruginosa* strain selected from the group consisting of Deposit Accession Nos. KCTC 12901 BP, KCTC 12902BP, KCTC 12903BP, and KCTC 12904BP.

The attenuated *P. aeruginosa* strain according to the present disclosure is a novel safe *P. aeruginosa* strain that exhibits improved productivity and no toxicity of *P. aeruginosa* itself and is attenuated to allow its cell wall protein to exhibit only antigenicity. In an embodiment of the present disclosure, it was confirmed by cell mass measurement that productivity of the novel *P. aeruginosa* strain is improved as compared with the known *P. aeruginosa* strain (Table 1).

The productivity improvement of *P. aeruginosa* strain is important in that it is directly related to productivity increase in the preparation of live vaccines and killed vaccines, or it is directly related to a recovery rate when a product derived from the bacterial strain is used.

*P. aeruginosa* is classified into type 1 to type 7 according to Fisher-Devlin immunotype. In particular, the strains of type 1 and type 3 among the 7 types of *P. aeruginosa* strain are most frequently detected in *P. aeruginosa*-infected patients, and the present disclosure relates to types 1, 2, 3, and 6 of *P. aeruginosa* strains which are most frequently detected.

Each type of *P. aeruginosa* strain produces many extracellular substances which play an important role as pathogenic factors. Lipopolysaccharides, exotoxin A, protease, elastase, lecithinase, collagenase, glycolipid, surface slime, leukocidin, enterotoxin, pigments, and exoenzyme etc. are reported as the pathogenic factors of *P. aeruginosa.* Among them, protease, together with endotoxin and exotoxin, is produced in most clinical isolates, and is known as a highly toxic substance next to exotoxin (Liu PV, Extracellular toxins. 132:S94, 1974).

The term "attenuated", as used herein, means that a toxic strain is modified to a less toxic strain or a weak pathogenic strain, as compared with the strain before modification, and this strain has remarkably reduced ability to induce clinical diseases during replication and division in a host. The attenuated strain of the present disclosure may a strain of which toxicity or pathogenicity becomes as low as acceptable for vaccination, and may be also a strain that is attenuated not to induce clinical diseases while maintaining replication in a host.

The attenuated strain according to the present disclosure is obtained by improving productivity and safety through repeated attenuation of *P. aeruginosa* strain with Deposit Accession No. KCCM 10029, KCCM 10030, KCCM 10031, or KCCM 10034 as a parent strain. The novel attenuated *P. aeruginosa* strain thus obtained was designated as EGPA01, EGPA02, EGPA03, or EGPA06 according to the fisher type of the parent strain, i.e., KCCM 10029 (fisher type 1), KCCM 10030(fisher type 2), KCCM 10031 (fisher type 3), or KCCM 10034 (fisher type 6), respectively.

The attenuated *P. aeruginosa* strain obtained through the repeated attenuation is almost identical to the existing *P. aeruginosa* strains in morphology and mycological properties, but has considerably improved productivity and safety, as compared with *P. aeruginosa* strain, and therefore, the attenuated *P. aeruginosa* strain is classified as a novel strain. Accordingly, these strains were deposited in the Korean Collection for Type Cultures (KCTC), which is an international depository authority, under Deposit Accession Nos. KCTC 12901BP for EGPA01, KCTC 12902BP for EGPA02, KCTC 12903BP for EGPA03, and KCTC 12904BP for EGPA06 on September 22, 2015.

Accordingly, in the present disclosure, Deposit Accession Nos. KCTC 12901BP, KCTC 12902BP, KCTC 12903BP, and KCTC 12904BP may be obtained by attenuating Deposit Accession Nos. KCCM 10029, KCCM 10030, KCCM 10031, and KCCM 10034, respectively, but are not limited thereto.

In another aspect, the present disclosure relates to a method of preparing the attenuated *P. aeruginosa* strain, the method including repeatedly subculturing 40 to 60 times a strain selected from the group consisting of Deposit Accession Nos. KCCM 10029, KCCM 10030, KCCM 10031, and KCCM 10034.

The preparation method may include, for example, (a) selecting a colony from colonies obtained by culturing the *P. aeruginosa* strain, wherein i) the colony's surface is not smooth, ii) a clear zone is not formed around the colony or a clear zone is less formed than those of other colonies, or iii) the colony's size is larger than those of other colonies; and (b) repeatedly subculturing the selected colony 40 to 60 times.

The preparation method of the present disclosure includes procedures of selecting a particular colony from colonies produced by culturing *P. aeruginosa* strain and performing repeated subculturing of the selected colony. The configuration included in the corresponding procedures overlaps with the configuration included in the description of the attenuated *P. aeruginosa* strain mentioned above, and thus the same description is applied thereto.

According to an embodiment of the present disclosure, *P. aeruginosa* strain was streaked and cultured on a tryptic-soy broth (TSB) agar plate containing skimmed milk, and as a result, colonies were produced. From the produced colonies, a colony was selected, wherein the colony's surface is not smooth, its size is large, and a clear zone around the colony is less formed than those of other colonies.

When the selected colony was repeatedly cultured for 40 passages or more, for example, 40 passages to 50 passages, or 40 passages to 60 passages, the desired attenuated *P. aeruginosa* strain may be obtained. When the selected colony was cultured for less than 40 passages, it is difficult to obtain the *P. aeruginosa* strain which is attenuated at a desired degree. When the selected colony was cultured for more than 60 passages, attenuation does not further occur and properties of the bacterium may be transformed.

In still another aspect, the present disclosure relates to a vaccine composition for preventing *P. aeruginosa* infection, the vaccine composition including the attenuated *P. aeruginosa* strain.

The term "vaccine", as used herein, refers to an antigen used for immunization to prevent infectious diseases, which is prepared as an attenuated microorganism or virus, and the vaccine means that a subject is administered with a pathogenic microorganism which is attenuated or killed in order to artificially acquire immunogenicity against particular infection. When stimulated by the vaccine, the immune system in the subject is activated to generate antibodies. The sensitization is maintained, when reinfection occurs, the antibodies may be effectively generated within a short time, thereby overcoming the disease.

The vaccine including the attenuated *P. aeruginosa* strain effectively induces formation of antibodies against *P. aeruginosa* strain when administered into the human body, without risk of inherent toxicity of *P. aeruginosa* itself, thereby exhibiting excellent immunogenic reaction. In particular, a cell wall protein of *P. aeruginosa* is useful in the preparation of prophylactic vaccines for *P. aeruginosa* infection, and it is possible to prepare a therapeutic agent for *P. aeruginosa* infection including, as an active ingredient, human immunoglobulins specifically binding to outer membrane proteins which are isolated and purified from *P. aeruginosa* strain.

In the present disclosure, the vaccine composition for preventing *P. aeruginosa* infection may include one or more attenuated *P. aeruginosa* strains among the above attenuated *P. aeruginosa* strains. In an embodiment of the present disclosure, the vaccine composition may essentially include Deposit Accession No. KCTC 12901 BP, for example, i) Deposit Accession No. KCTC 12901 BP and iii) any one selected from the group consisting of KCTC 12902BP, KCTC 12903BP, and KCTC 12904BP. In one or more embodiments of the present disclosure, the vaccine composition may include KCTC 12901 BP and KCTC 12902BP; KCTC 12901 BP and KCTC 12903BP; KCTC 12901BP and KCTC 12904BP; KCTC 12901BP, KCTC 12902BP, and KCTC 12903BP; KCTC 12901BP, KCTC 12903BP, and KCTC 12904BP; KCTC 12901BP, KCTC 12902BP, KCTC 12903BP, and KCTC 12904BP; KCTC 12902BP and KCTC 12903BP; KCTC 12902BP and KCTC 12904BP; KCTC 12903BP and KCTC 12904BP; or KCTC 12902BP, KCTC 12903BP, and KCTC 12904BP.

The subject to which the vaccine of the present disclosure may be administered is not limited, and may include mammals such as rats, mice, livestock, and humans, etc.

An effective administration dose range of the vaccine of the present disclosure may vary depending on various factors well known in the medical field, such as sex, body surface area, kind and severity of a disease, age, drug sensitivity, administration route and excretion rate, administration time, treatment period, target cells, expression level, etc., and may be easily determined by those skilled in the art.

The vaccine composition of the present disclosure may further include a pharmaceutically acceptable carrier or adjuvant, in addition to the attenuated *P. aeruginosa* strain.

The carrier which may be included in the composition of the present disclosure may include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxybenzoate, propyl hydroxylbenzoate, talc, magnesium stearate, mineral oil, etc.

Further, the adjuvant which may be used in the present disclosure may be any adjuvant commonly used in the art without limitation. In an embodiment of the present disclosure, the adjuvant may be cholera toxin binding unit, aluminum salts, an lipid emulsion (MF-59), a synthetic detergent (Tween), microspheres, liposomes, mucosa adhesive polymers, etc., but is not limited thereto. As new formulations of this field are being developed, they may also be used.

The composition according to the present disclosure may be used after being formulated according to a common method in oral dosage forms, including powders, granules, tablets, capsules, suspensions, emulsions, syrup, aerosol, etc., preparations for external application, suppositories, or sterile injectable solutions.

Specifically, upon formulation, commonly used diluents or excipients, such as fillers, extenders, binders, wetting agents, disintegrants, surfactants, etc. may be used. Solid formulations for oral administration may include tablets, pills, powders, granules, capsules, etc., and such solid formulations may be prepared by mixing the composition with at least one excipient, for example, starch, calcium carbonate, sucrose, lactose, gelatin, etc. In addition to simple excipients, lubricants such as magnesium stearate or talc may also be used. Liquid formulations for oral administration may include suspensions, solutions for internal use, emulsions, syrup, etc., and may include various excipients, for example, wetting agents, flavoring agents, aromatics, preservatives, etc., in addition to water and liquid paraffin, which are simple diluents frequently used. Formulations for parenteral administration may include sterilized aqueous solutions, non-aqueous solutions, suspensions, emulsions, lyophilized preparations, suppositories, etc. As non-aqueous solvents or suspending agents, propylene glycol, polyethylene glycol, vegetable oils such as olive oil, injectable esters such as ethyl oleate, etc. may be used. As a base of the suppositories, witepsol, Macrogol, Tween 61, cacao butter, laurin butter, glycerogelatin, etc. may be used.

Examples of the *P. aeruginosa* infection to which the vaccine composition of the present disclosure is applied may include sepsis, systemic infection, chronic infection of respiratory tract, cystic fibrosis, etc., but are not limited thereto.

### MODE OF DISCLOSURE

Hereinafter, the present disclosure will be described in more detail with reference to Examples. However, it is apparent to those skilled in the art that these Examples are for illustrative purposes only, and the scope of the present disclosure is not intended to be limited by these Examples.

### Example 1: Comparison of Production Yields (Wet Cell Mass) after Culturing Parent Strains and Attenuated Strains of Pseudomonas aeruginosa

In order to compare production yields between *P. aeruginosa* strain and attenuated strains of the present disclosure, each of the test strains was streaked on a TSB agar plate and cultured in a 37°C incubator for 16 hours to 20 hours. Each of test strain colonies was taken and seeded in a test tube containing 10 ml TSB, and cultured at 37°C and 200 rpm for 16 hours to 20 hours. 1 ml of the culture was taken and seeded in 10 ml of fresh TSB, and cultured for 6 hours to 8 hours. It was examined whether optical density of the culture was OD₆₀₀>1.0, and then the culture was diluted to adjust OD₆₀₀ at 1.0. After optical density was uniformly adjusted at OD₆₀₀=1.0, 5 ml of each culture was seeded in a 500 ml flask containing 100 ml of TSB to adjust a seed volume at 5% (v/v). Culturing was performed at 37°C, 200 rpm for 8 hours to 10 hours. After completing the culturing, each cell culture was centrifuged under conditions of 7000 rpm, 20 minutes, and 4°C, and a supernatant was discarded, and weights of wet cells were measured.

As a result, as shown in Table 1, when the measured weights of the wet cells were compared, the attenuated strains of the present disclosure showed a yield increase of 112% to 131%, as compared with known *P. aeruginosa* strains, indicating productivity improvement. In particular, EGPA06 of Fisher type 6 showed remarkably increased productivity of 131%, as compared with KCCM 10034 of the same type.

**[Table 1]**

| | Wet cell (g) | | Yield increase |
|---|---|---|---|
| | Parent strain | Attenuated strain | |
| Fisher type 1 | 1.28 | 1.61 | 125.8% |
| Fisher type 2 | 1.57 | 1.77 | 112.7% |
| Fisher type 3 | 1.67 | 2.02 | 130% |
| Fisher type 6 | 1.58 | 2.07 | 131% |

### Example 2: Comparison of Cytotoxicity of Parent Strains and Attenuated Strains of P. aeruginosa

In order to compare cytotoxicity of *P. aeruginosa* strain and the attenuated strain of the present disclosure, experiments including the following procedures were performed. WST assay was performed by using an EZ-Cytox cell viability assay kit (Daeil Lab science, Korea).

### A. Cell Culture and Seeding

BEAS-2B cells frozen in liquid nitrogen were taken and thawed in a water bath at 37°C for about 3 minutes. The thawed cells were transferred to a 15 ml conical tube and then 9 ml of complement medium was added thereto, followed by centrifugation at 1,300 rpm, 4°C for 5 minutes. After a supernatant was discarded, 1 ml of complement medium was added to suspend a cell pellet. 1) The suspension was transferred to 10 ml of complement medium in a 75Φ cell culture flask and cultured in a 5% CO₂ incubator at 37°C. 2) When cells were grown to about 70% ∼ about 80% of the area of the 75Φ cell culture flask, the cells were detached with 3 ml of trypsin-EDTA, and recovered in a 15 ml conical tube. 3) Centrifugation was performed at 1,300 rpm, 4°C for 5 minutes. 4) A supernatant was discarded and 5 ml of fresh complement medium was added to suspend a cell pellet. 5) 20 µl of the suspended cells was taken and diluted and stained with a trypan blue solution at a predetermined ratio. 6) Complement medium and 10 ml of the suspended cells were added to a 75Φ cell culture plate at a cell density of 1×10⁶ cells/ml. Every 2 days, the procedures of 1) to 6) were performed to maintain the cells until assay. In a clean bench, cells attached on the plate were detached with 3 ml of trypsin-EDTA and then recovered in a 15 ml conical tube, which was filled with complement medium to 10 ml. Centrifugation was performed at 1,300 rpm, 4°C for 5 minutes. A cell pellet was remained and 1 ml of fresh medium was added thereto to suspend the cell pellet. 20 µl of the suspended cells was taken and diluted and stained with a trypan blue solution at a predetermined ratio. 10 µl of the stained cells were added to a hemocytometer, and the number of cells were counted under a microscope. After the cells were prepared at a density of 4×10⁴ cells/ml, 100 µl/well was provided to a 96-well cell culture plate. In this regard, 100 µl/well of a background control (cell-containing culture medium+Ez-cytox) was also seeded. The cells were cultured in a CO₂ incubator for 24 hours.

### B. Sample Treatment

5 ml of TSB medium was added to a glass tube, and 10 µl of *P. aeruginosa* strain (stock) was seeded thereto, followed by culturing at 37°C, 200 rpm for 16 hours. After OD₆₀₀ of the *P. aeruginosa* culture was measured, the culture was diluted with TSB at OD₆₀₀ of 0.6, and 500 µl thereof was seeded in a glass tube containing 5 ml of TSB medium. After culturing at 37°C, 200 rpm for 2 hours and 30 minutes, 5 ml of the culture was centrifuged at 7,000 rpm, RT for 5 minutes. Washing was performed with 5 ml of PBS three times. After suspending with 3 ml of PBS, OD was measured. The cells were diluted with PBS at final OD of 0.6, and then diluted at 1×10⁶ CFU/ml or 5×10⁶ CFU/ml. After removing the medium from the 96-well culture plate, 100 µl of DMEM without FBS, penicillin and streptomycin was added to each well. In this regard, 100 µl of DMEM for background controls (cell-free culture medium+Ez-cytox, and cell-free culture medium+*P. aeruginosa*+Ez-cytox)) was also added to each well. 10 µl of the diluted *P. aeruginosa* strain was added to each well of 96-well plate containing cells, and cultured in a 5% CO₂ incubator at 37°C for 24 hours.

### C. Cytotoxicity (WST) Assay

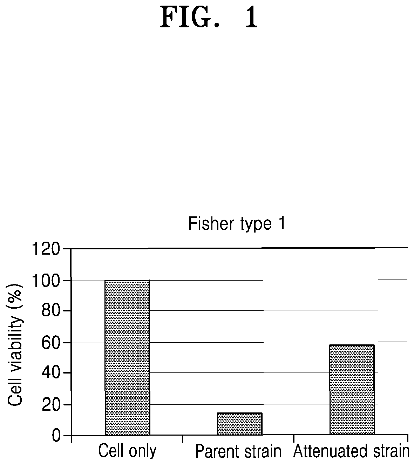

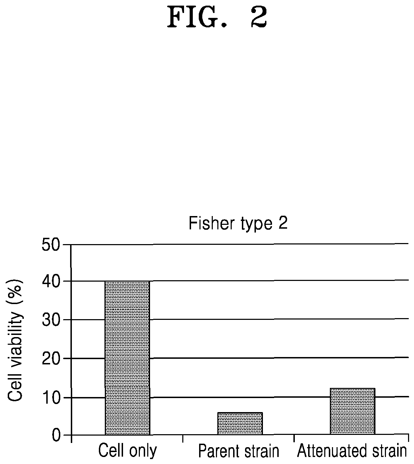

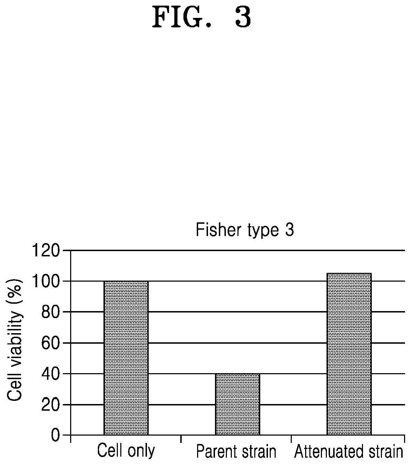

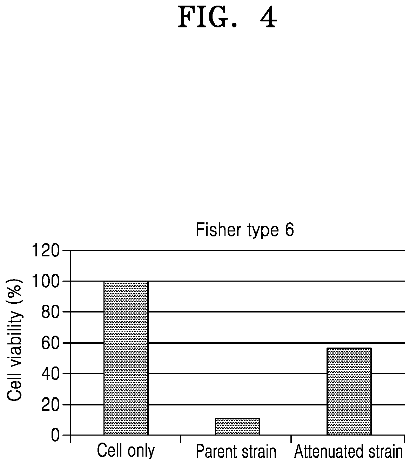

The 96-well culture plate cultured for 24 hours was washed with PBS three times. After washing, 100 µl of DMEM without FBS, penicillin and streptomycin was added to each well of the 96-well culture plate. 10 µl of EZ-Cytox was added to each well, and allowed to react in an incubator for 30 minutes. The plate were gently shaken for about 1 minute before measuring optical density. Optical density at 450 nm was measured by using a plate reader (FIGS. 1 to 4).

As a result, as shown in FIGS. 1 to 4, the attenuated strains of the present disclosure showed increased viability in all cell types, as compared with *P. aeruginosa* strain.

While the present disclosure has been particularly described, it will be apparent to those skilled in the art that the detailed description is merely as exemplifications of preferred embodiments, and the scope of the present disclosure is not limited thereto. Therefore, the scope of the present disclosure is defined by the appended claims and equivalents thereof.

### [Deposit Accession Number]

1. Name of depository institution: Korean Collection for Type Cultures
   Deposit Accession number: KCTC 12901BP
   Deposit date: 2015. 9. 22
2. Name of depository institution: Korean Collection for Type Cultures
   Deposit Accession number: KCTC 12902BP
   Deposit date: 2015. 9. 22
3. Name of depository institution: Korean Collection for Type Cultures
   Deposit Accession number: KCTC 12903BP
   Deposit date: 2015. 9. 22
4. Name of depository institution: Korean Collection for Type Cultures
   Deposit Accession number: KCTC 12904BP
   Deposit date: 2015. 9. 22

### INDUSTRIAL APPLICABILITY

According to the present disclosure, it is possible to prepare a novel vaccine which is improved as compared with existing vaccines for *P. aeruginosa* infection, by improving productivity and safety through remarkably reduced cytotoxicity due to reduced release of pathogenic factors, as compared with known attenuated *P. aeruginosa* strains.

## Claims

1. An attenuated *Pseudomonas aeruginosa* strain selected from the group consisting of Deposit Accession Nos. KCTC 12901 BP, KCTC 12902BP, KCTC 12903BP, and KCTC 12904BP.

2. The attenuated *Pseudomonas aeruginosa* strain of claim 1, wherein Deposit Accession Nos. KCTC 12901 BP, KCTC 12902BP, KCTC 12903BP, and KCTC 12904BP are obtained by attenuating Deposit Accession Nos. KCCM 10029, KCCM 10030, KCCM 10031, and KCCM 10034, respectively.

3. A vaccine composition for preventing *Pseudomonas aeruginosa* infection, the vaccine composition comprising one or more attenuated *Pseudomonas aeruginosa* strains selected from the group consisting of Deposit Accession Nos. KCTC 12901BP, KCTC 12902BP, KCTC 12903BP, and KCTC 12904BP.

4. The vaccine composition of claim 3, comprising Deposit Accession No. KCTC 12901BP; and one or more attenuated *Pseudomonas aeruginosa* strains selected from the group consisting of Deposit Accession Nos. KCTC 12901 BP, KCTC 12902BP, KCTC 12903BP, and KCTC 12904BP.

5. The vaccine composition of claim 3, comprising KCTC 12901BP and KCTC 12902BP; KCTC 12901BP and KCTC 12903BP; KCTC 12901BP and KCTC 12904BP; KCTC 12901BP, KCTC 12902BP, and KCTC 12903BP; KCTC 12901BP, KCTC 12903BP, and KCTC 12904BP; KCTC 12901BP, KCTC 12902BP, KCTC 12903BP, and KCTC 12904BP; KCTC 12902BP and KCTC 12903BP; KCTC 12902BP and KCTC 12904BP; KCTC 12903BP and KCTC 12904BP; or KCTC 12902BP, KCTC 12903BP, and KCTC 12904BP.

6. A method of preparing the attenuated *Pseudomonas aeruginosa* stain of claim 1, the method comprising repeatedly subculturing 40 to 60 times a strain selected from the group consisting of Deposit Accession Nos. KCCM 10029, KCCM 10030, KCCM 10031, and KCCM 10034.

7. The method of claim 6, further comprising:
(a) selecting a colony from colonies obtained by culturing *Pseudomonas aeruginosa,* wherein i) the colony's surface is not smooth, ii) a clear zone is not formed around the colony or a clear zone is less formed than those of other colonies, or iii) the colony's size is larger than those of other colonies; and
(b) repeatedly subculturing the selected colony 40 to 60 times.
